# EUROPEAN PATENT SPECIFICATION

(11) **EP 1 090 642 B1**
(45) Date of publication and mention of the grant of the patent: **09.07.2008**
(21) Application number: 00203772.9
(22) Date of filing: 04.06.1996
(51) Int. Cl.: A61K 39/385, A61K 39/116, A61K 39/295, A61K 39/00, A61P 31/00

(54) **Vaccines comprising a polysaccharide antigen-carrier protein conjugate and free carrier protein**
Vakzine mit einem Polysaccharide Antigen-Trägerprotein Konjugat und freiem Trägerprotein
Vaccins comprenant un conjugué antigènique de polysaccharide-protéine porteuse et une protéine porteuse libre

(30) Priority: 07.06.1995 US 472639; 23.06.1995 GB 9512827; 01.07.1995 GB 9513443; 15.12.1995 GB 9525657
(43) Date of publication of application: 11.04.2001
(62) Divisional of application: 96920790.1
(73) Proprietor: GlaxoSmithKline Biologicals s.a., 1330 Rixensart (BE)
(72) Inventor: Slaoui, Moncef Mohamed, SmithKline Beecham Biol.SA, 1330 Rixensart (BE); Hauser, Pierre, SmithKline Beecham Biologicals S.A, 1330 Rixensart (BE)
(74) Representative: Privett, Kathryn Louise

(56) References cited:
- EP-A- 0 208 375
- EP-A- 0 594 950
- US-A- 4 644 059
- BARINGTON, T. ET AL.: "Opposite effects of actively and passively acquired immunity to the carrier on responses of human infants to a Haemophilus influenzae type b conjugate vaccine" INFECTION AND IMMUNITY, vol. 62, 1994, pages 9-14, XP000602292
- CORBEL, M.J.: "Control testing of combined vaccines: a consideration of potential problems and approches" BIOLOGICALS, vol. 22, 1994, pages 353-360, XP000603003
- B¹GU¹, P.: "Vaccins haemophilus influenzae" BULLETIN DE L'ACAD¹MIE NATIONALE DE M¹DECINE, vol. 177, 1993, pages 1381-1387, XP000602905
- BARINGTON, T. ET AL.: "Non-epitope-specific suppression of the antibody response to Haemophilus influenzae type b conjugate vaccines by preimmunization with vaccine components" INFECTION AND IMMUNITY, vol. 61, 1993, pages 432-438, XP000602293
- FRASCH, C.E.: "Haemophilus influenzae type b conjugate and combination vaccines" CLINICAL IMMUNOTHERAPEUTICS, vol. 4, November 1995 (1995-11), pages 376-386, XP000602898

## Description

The present invention relates to combination vaccines comprising a conjugated polysaccharide antigen linked to a carrier protein, and wherein the carrier protein is also present as a free antigen in the vaccine composition. In particular the vaccine composition of the present invention relates to a multivalent vaccine, that is a vaccine for the amelioration or treatment of more than one disease states. The present invention also relates to the production and use of such a vaccine in medicine.

Vaccines that utilise polysaccharides are known in the art. For example a vaccine for the prevention of Haemophilus influenzae b (Hib) infections are based on the capsular polysaccharide (PRP) conjugated with a carrier protein. Typically the carrier protein is a diphtheria or tetanus toxoid. It has also been suggested to produce a vaccine for the prevention of Streptococcus pneumonia which is based on capsular polysaccharide conjugated to a carrier protein such as tetanus toxoid or diphtheria toxoid. Examples of such conjugate vaccine antigens are disclosed in US 4 365 170 US 4 673 574 EP 208 375 EP 477508 and EP 161 188.

It is desirable to administer such conjugate vaccines with other antigens or vaccines at the same time and this can involve multiple injections. Problems associated with multiple injections include a more complicated administration procedure and a large total injection volume. This is a particularly acute problem when the vaccine is intended for infants.

It has therefore been proposed to produce combination vaccines. One well known combination vaccine provides protection against Diphtheria, tetanus and B. pertussis infections. This vaccine comprises a pertussis component (either killed whole cell B. pertussis or accellular pertussis which typically consists of up to three antigens - PT, FHA and 69kDa) and toxoided diphtheria and tetanus toxin. Such vaccines are often referred to as DTPw (whole cell) or DTPa (accellular).

It would be desirable to add polysaccharide conjugate vaccines to such a combination. However we have found that simple mixing of the components results in a reduction of antibody titres to the polysaccharide component and that the persistence of protective levels of such antibody titres is reduced. Thus the immunogenicity of conjugate PRP Hib polysaccharide, to detoxified Tetanus Toxoid (TT) has been observed to decrease in infants immunised with TT-PRP conjugate combined to DTPa as compared to infants immunised with TT-PRP conjugate and DTPa concomitantly, but at separate sites.

In the monovalent situation, ie when a vaccine comprises just a single polysaccharide (PS) conjugated to a carrier protein, the higher the protein content of the conjugate the higher the antibody response to the polysaccharide. This is thought to be because induction of a T cell dependent polysaccharide specific antibody response requires the covalent coupling of a protein carrier to the polysaccharide. The protein carrier needs to be present at concentrations (ie PS to protein ratio) such that a good TH cell response to the carrier is induced, and that this TH response can recognise PS specific B cell clones expressing peptides derived from the covalently coupled carrier protein.

Typically the prior art monovalent vaccines have a ratio of polysaccharide: protein carrier of 1:3 (weight:weight).

Surprisingly and in complete contrast to the position in the monovalent vaccine, the present inventors have discovered that when the polysaccharide conjugate component is part of a multivalent vaccine comprising a free form of the carrier protein, the immunogenicity of the polysaccharide component is improved by reducing the protein content of the conjugate antigen.

Accordingly the present invention provides a combined vaccine of claim 1. A preferred ratio is 1:0.5 to 1:1.5, typically in the order of 1:1.

The polysaccharide conjugate may be prepared by any known coupling technique. For example the polysaccharide can be coupled via a thioether linkage. This conjugation method relies on activation of the polysaccharide with 1-cyano-4-dimethylamino pyridinium tetrafluoroborate (CDAP) to form a cyanate ester. The activated polysaccharide may thus be coupled directly or via a spacer group to an amino group on the carrier protein. Preferably, the cyanate ester is coupled with hexane diamine and the amino-derivatised polysaccharide is conjugated to the carrier protein using heteroligation chemistry involving the formation of the thioether linkage. Such conjugates are described in PCT published application WO93/15760 Uniformed Services University.

The conjugates can also be prepared by direct reductive amination methods as described in US 4365170 (Jennings) and US 4673574 (Anderson). Other methods are described in EP-0-161-188, EP-208375 and EP-0-477508.

A further method involves the coupling of a cyanogen bromide activated polysaccharide derivatised with adipic acid hydrazide (ADH) to the protein carrier by Carbodiimide condensation (Chu C. et al Infect. Immunity, 1983 245 256).

Preferably the carrier protein is an antigen derived from a Tetanus, or Diphtheria preferably tetanus toxoid or Diphtheria toxoid. The carrier may also be derived from Bordetella. Typically the antigen is non toxic derivative of a toxin from Tetanus or Diphtheria.

Preferably the compositions of the invention contain a suitable adjuvant.

A preferred adjuvant is MPL (3-deacylated monophosphoryl lipid A, also known as 3D-MPL). 3D-MPL is known from GB 2 220 211 (Ribi) as a mixture of 3 De-O-acylated monophosphoryl lipid A with 4, 5 or 6 acylated chains and is manufactured by Ribi Immunochem, Montana. A preferred form of MPL is disclosed in International Patent Application 92/116556.

Another adjuvant which may be used in the present invention is known as QS21. QS21 is a Hplc purified non toxic fraction of a saponin from the bark of the South American tree Quillaja saponaria molina and its method of its production is disclosed (as QA21) in US patent No. 5,057,540.

Combinations of QS21 and 3 D-MPL are known to produce a synergistically effective vaccine formulation and are described in International Patent application WO 94/00153.

In some cases it may be that the entire vaccine of the present invention will be presented in an oil in water emulsion, or other suitable vehicle, such as for example, liposomes, microspheres or encapsulated antigen particles.

The vaccine formulations may contain further antigens. Antigen or antigenic compositions known in the art can be used in the compositions of the invention, including antigen or antigenic compositions derived from HIV-1, (such as gp120 or gp160), human or animal herpes viruses, such as gD or derivatives thereof or Immediate Early protein such as ICP27 from HSV1 or HSV2, cytomegalovirus (especially human)(such as gB or derivatives thereof), Varicella Zoster Virus (such as gpI, II or III), or from a hepatitis virus such as hepatitis B virus for example Hepatitis B Surface antigen or a derivative thereof, hepatitis A virus, hepatitis C virus and hepatitis E virus, or from other viral pathogens, such as Respiratory Syncytial virus, human papilloma virus or Influenza virus, or polio virus such as Inactivated Polio Virus (IPV) or derived from bacterial pathogens such as Diphtheria (D), Tetanus (T), Salmonella, Neisseria, particularly Neisseria Meningitis A, B, or C. Borrelia (for example OspA or OspB or derivatives thereof), or Chlamydia, or Bordetella for example P.69, PT and FHA from B.pertussis (P), or derived from parasites such as plasmodium or Toxoplasma.

The above combinations may optionally include a component which is protective against Hepatitis A.

Suitable components for use in such vaccines are already commercially available and details may be obtained from the World Health Organisation. For example the IPV component may be the Salk inactivated polio vaccine. The Diphtheria, Tetanus and Pertussis vaccine may comprise an accellular product such as Infanrix DTPa (SmithKline Beecham Biologicals). The component affording protection against Hepatitis A is preferably the product known as 'Havrix' (SmithKline Beecham Biologicals) which is a killed attenuated vaccine derived from the HM-175 strain of HAV [see 'Inactivated Candidate Vaccines for Hepatitis A' by F.E. Andre, A Hepburn and E.D'Hondt, Prog Med. Virol. Vol 37, pages 72-95 (1990) and the product monograph 'Havrix' published by SmithKline Beecham Biologicals (1991)]. The Hepatitis B component may comprise the 'S' antigen as in 'Engerix-B'.

Advantageously the combination vaccine according to the invention is a paediatric vaccine.

Vaccine preparation is generally described in Vaccine Design - the subunit and Adjuvant approach edited by Michael F Powell and Mark Newman, Plenum Press. Encapsulation within liposomes is described, for example, by Fullerton, US Patent 4,235,877. Conjugation of proteins to macromolecules is disclosed, for example, by Likhite, US Patent 4,372,945 and by Armor et al., US Patent 4,474,757.

The amount of antigen in each vaccine dose is selected as an amount which induces an immunoprotective response without significant, adverse side effects in typical vaccinees. Such amount will vary depending on which specific immunogens are employed. Generally it is expected that each dose will comprise 1-1000ug of total immunogen, preferably 2-100ug, most preferably 4-40ug. An optimal amount for a particular vaccine can be ascertained by standard studies involving observation of antibody titres and other responses in subjects. Following an initial vaccination, subjects may receive one or two booster injections at about 4 weeks intervals.

The following examples will illustrate the invention.

### Examples

### Example 1a) Vaccine comprising DTPa - Hib PRP-TT - and HBsAg.

### Production of Hib TT conjugate

### Materials and Methods

### Materials

The Hib PRP is extracted and purified from inactivated cell cultures. The purified material meets the WHO and US specifications in terms of residual protein, nucleic acid, endotoxin, structural sugars and molecular size distribution.

Tetanus toxoid produced by Behringwerke meets also the WHO specifications. The material is further purified by acid pH precipitation and gel filtration chromatography to isolate the monomeric form (150 kDa) of TT.

### Activation and Coupling Chemistry

20 mg of Hib PRP were dissolved in 4 ml of 2 M Na Cl.

150 mcl of CDAP (1-cyano-4-dimethylamino-pyridinium tetrafluoroborate) was added to the polysaccharide solution (from a 100 mg/ml stock solution in acetronitrile). One minute later, 300 mcl of triethylamine 0.2 M was added. The activation of the polysaccharide was performed during 2 minutes at 0°C at pH 9.5-10.

20 mg or 40 mg of the Tetanus toxoid (initial PRP/Protein ratio of 1/1 or 1/2) at a concentration of 15 mg/ml was added and the coupling reaction was performed at 25°C for 1 hour. Then the reaction was quenched for 1 hour at 25°C with 3 ml of 1 M glycine solution pH 5.0 and then overnight at 4°C.

The conjugate is purified by gel filtration chromatography using a Sephacryl HR 500 gel filtration column equilibrated with 0.2 M NaCl. The carbohydrate and protein content of the eluted fractions was determined. The conjugate was pooled in and sterile filtered. The PRP/protein ratio and free PS content in the conjugate preparation were determined.

### Example 1b)

### DTP Hib PRP TT HBsAg Combination Vaccine Formulation

Formulation of DTPa (comprising Diphtheria toxoid, Tetanus toxoid, pertussis toxoid, pertactin and FHA) with or without Hepatitis B vaccines are known in the art. For example such a vaccine is disclosed in International Patent application No WO 93/24148. The Conjugate as described in example 1 was added to the vaccine and mixed prior to injection for immunogenicity studies.

In the present studies the following steps were undertaken:
- adjustment of phenoxy-ethanol (bacteriostatic) content to 5 mg/ml
- adjustment of pH at 6.1 ± 0.1
- adjustment of PRP concentration up to 100 mcg/ml
- addition of DTPaHBV (0.5 ml) to 0.1 ml of the PRP-CDAP-TT solution.

The final composition of the vaccine was for one dose:
- PRP'P 10 mcg
- D 25 Lf
- T 10 Lf
- PT 25 mcg
- FHA 25 mcg
- 69K 8 mcg
- Hep B 10 mcg
- Al (OH)₃ Behring 0.35 mg
- Al (OH)₃ Superpos 0.15 mg
- AlPO₄ Superpos 0.20 mg
- NaCl 150 mM
- 2ϕEt-OH 3 mg (→5 mg/ml)
- pH 6.1 ± 0.1
- Vol 0.6 ml

### Example 2

### Immunogenicity

The immunogenicity of Haemophilus Influenzae type B derived polysaccharide:protein (weight:weight) ratios (1:3, 1:2 and 1:1), using two different coupling chemistries, is compared after a first and second inoculation in young OFA rats.

The data obtained at 28 days after the first vaccine inoculation clearly show an improved anti PS antibody titre correlating with a decreased protein content in the conjugate vaccine, when the conjugate antigen is combined to DTPa Hepatitis B, but not when the conjugate is injected alone. This is in clear contrast to the situation observed when the conjugate vaccine is given alone, where the Lower PSTT ratio (ie more carrier protein) results in a better antibody response to the Polysaccharide (see day 42 anti PS titre of Groups 1 to 3 going from 25, to 18 to 5 µg/ml with a decreasing amount of TT, while they go from .3, 4 to 8 to 19 µg/ml for the same TT contents, but when given in combination with DTPa He B vaccine.

### Example 3

### Effect of TT/PS ratio on compatibility of Hib with DTPa

Groups of 10 rats (OFA, 5 weeks of age, female) were immunised 2 times at 24 days interval by the subcutaneous route with the different TT-PRP vaccines given at 2 dosages: 0.5γ PS/rat (table 2) or 0.05γ PS/rat (table 3). The Hib001 vaccine was reconstituted with saline or 1 dose of DTPa (lot 119) to have a concentration of 10γ PS/ml. The liquid vaccines were diluted with saline or 1 human dose of DTPa to have 10γ PS/ml. The vaccines were then further diluted in saline to have 2.5γ PS/ml or 0.25γ PS/ml. The rats were injected with 200 µl of each vaccine within one hour after dilution.

The animals were bled at day 24 and day 38 and the anti-PRP IgG Abs were measured by ELISA in individual sera and expressed in γ/ml. The GMT was calculated for each group.

### Example 4

### Comparison of Immunogenicity of DTPa, and DTPa Hepatitis B vaccine mixed with low ratio or 'normal' PRP:TT

Groups of 10 rats (OFA, 1 week of age) were immunised three times (SC) at two week intervals with 0.5µg of PS combined 1 hour before injection with 1/20th a human dose of DTPa or DTPa HepB. The animals were bled 2 weeks after the third dose and the antibodies against pertussis toxoid (PT), FHA, pertactin (69k), Diphtheria toxoid (D) Tetanus toxoid (T) and Hepatitis B surface antigen (HBS) measured by Elisa. The titres, expressed as a mid point dilution using a reference are shown in Table 4.

The results of this experiment together with the result from example 3 demonstrate that the Hib response is enhanced in a combination vaccine, if the amount of protein in the conjugate is reduced. Moreover, there is no significant interference to the immune response of any other component of the vaccine. *

**TABLE 1**

| **Conjugate** | **Ps/Protein Ratio Weight:Weight** | **Conjugate alone** | | **Conjugate plus Hep B and DTPa** | |
|---|---|---|---|---|---|
| | | **D28 (PI)** | **D42 (PII)** | **D28 (PI)** | **D42 (PII)** |
| Buffer | | 0.05 | 0.11 | 0.05 | 0.11 |
| | | | | | |
| PRP - TT | 1/3 | 0.5 | 25 | 3.4 | 29 |
| PRP-TT | 1/2 | 0.6 | 18 | 8 | 40 |
| PRP-TT | 1/1 | 0.3 | 5 | 19 | 38 |

**TABLE 2**

| **Vaccine** | **PS/TT** | ∝**-PRP (**γ/**ml)** | | | |
|---|---|---|---|---|---|
| | | **Alone** | | **+ DTPa** | |
| | | **d24** | **d38** | **d24** | **d38** |
| Nacl | - | 0.002 | 0.01 | 0.002 | 0.01 |
| Hib001 A44 (control) | 1/2.5 | 0.08 | 51.5 | 0.63 | 40.3 |
| Dhib015 | 1/3 | 0.88 | 64.1 | 0.47 | 19 |
| Dhib026 | 1/1 | 0.07 | 9.1 | 1.27 | 66.7 |

**TABLE3**

| **Vaccine** | **PS/TT** | ∝**-PRP Abs(**γ**/ml)** | | | |
|---|---|---|---|---|---|
| | | **Alone** | | **+ DTPa** | |
| | | **d24** | **d38** | **d24** | **d38** |
| Nacl | - | 0.002 | 0.01 | 0.002 | 0.01 |
| Hib001 A44 (control) | 1/2.5 | 0.05 | 7.0 | 0.09 | 14.4 |
| Dhib015 | 1/3 | 0.07 | 17.7 | 0.12 | 14.7 |
| Dhib026 | 1/1 | 0.02 | 0.45 | 0.17 | 51.6 |

**TABLE 4**

| **Vaccine** | **Ab response after 3 doses (d 42)** | | | | | | |
|---|---|---|---|---|---|---|---|
| | **∝-PRP** | **∝-D** | **∝-T** | **∝-PT** | **∝-FHA** | **∝-69k** | **∝-HBS** |
| None (NaCl) | <0.1 | <3 | <2 | <8 | <4 | <2 | <2 |
| DTPa + Hib | 3.4 | 2847 | 6976 | 1518 | 2119- | 127 | - |
| (Control PS/TT-1/2.5) | | | | | N | | |
| | | | | | S | | - |
| + Hib (low ratio) | 20.9 | 2847 | 9579 | 2144 | 3551- | 35 | |
| (PS/TT ^{~} 1/1) | | | | | | | |
| DTPaHB + Hib | 2.8 | 2001 | 3622 | 1603 | 1123- | 18 | 3622 |
| (PS/TT-1/2.5) | | | | | N | | |
| | | | | | S | | 6674 |
| + Hib (low ratio) | 11.4 | 1874 | 6674 | 1402 | 3035- | 12 | |
| (PS/TT - 1/1) | | | | | | | |

## Claims

1. A combined vaccine comprising:
a) a polysaccharide antigen derived from Streptococcus pneumoniae, or Neisseria meningitidis A, B or C conjugated to a carrier protein, and
b) the carrier protein also present as free antigen,
**characterised in that** the ratio of polysaccharide to carrier protein in the conjugate is from 1:0.3 to 1:2 (w/w).

2. A combined vaccine as claimed in claim 1 wherein the carrier protein is a Diphtheria or Tetanus antigen.

3. The combined vaccine of claim 2, wherein the Diphtheria or Tetanus antigen is Diphtheria toxoid or tetanus toxoid.

4. The combined vaccine of claim 2, wherein the Diphtheria or Tetanus antigen is a non toxic derivative of a toxin from Diphtheria or Tetanus.

5. A combined vaccine as claimed in any one of claims 1 to 4 wherein the ratio of polysaccharide: carrier protein is from 1:0.5 to 1:1.5 (w/w).

6. A combined vaccine as claimed in any one of claims 1 to 5, further comprising antigens protective against Diphtheria, Tetanus, and pertussis infection.

7. A combined vaccine as claimed in any one of claims 1 to 6 further comprising an antigen against hepatitis B virus.

8. A combined vaccine as claimed in any one of claims 1 to 7 further comprising an antigen against hepatitis A virus.

9. A combined vaccine as claimed in any one of claims 1 to 8 further comprising an antigen providing immunity against polio

10. A combined vaccine according to any one of clams 1 to 9 additionally comprising a suitable adjuvant.

11. A vaccine as claimed in any one of claims 1 to 10 for use in medicine.

12. A use of an effective amount of the combined vaccine as claimed in claims 1 to 10 in the manufacture of a medicament for the treatment of a human susceptible to or suffering from an infectious disease.

## Patentansprüche

1. Kombinierter Impfstoff, der folgendes umfaßt:
a) ein Polysaccharid-Antigen, das von Streptococcus pneumoniae oder Neisseria meningitidis A, B oder C stammt, konjugiert an ein Trägerprotein, und
b) das Trägerprotein, das ebenfalls als freies Antigen vorliegt,
**dadurch gekennzeichnet, daß** das Verhältnis von Polysaccharid zu Trägerprotein in dem Konjugat von 1:0,3 bis 1:2 (G/G) ist.

2. Kombinierter Impfstoff gemäß Anspruch 1, worin das Trägerprotein ein Diphtheria- oder Tetanus-Antigen ist.

3. Kombinierter Impfstoff gemäß Anspruch 2, worin das Diphtheria- oder Tetanus-Antigen Diphtheria-Toxoid oder Tetanus-Toxoid ist.

4. Kombinierter Impfstoff gemäß Anspruch 2, worin das Diphtheria- oder Tetanus-Antigen ein nicht-toxisches Derivat eines Toxins von Diphtheria oder Tetanus ist.

5. Kombinierter Impfstoff gemäß einem der Ansprüche 1 bis 4, worin das Verhältnis von Polysaccharid:Trägerprotein von 1:0,5 bis 1:1,5 (G/G) ist.

6. Kombinierter Impfstoff gemäß einem der Ansprüche 1 bis 5, der ferner Antigene umfaßt, die gegen Diphtheria-, Tetanus- und Pertussis-Infektion schützen.

7. Kombinierter Impfstoff gemäß einem der Ansprüche 1 bis 6, der ferner ein Antigen gegen Hepatitis-B-Virus umfaßt.

8. Kombinierter Impfstoff gemäß einem der Ansprüche 1 bis 7, der ferner ein Antigen gegen Hepatitis-A-Virus umfaßt.

9. Kombinierter Impfstoff gemäß einem der Ansprüche 1 bis 8, der ferner ein Antigen umfaßt, das eine Immunität gegen Polio verleiht.

10. Kombinierter Impfstoff gemäß einem der Ansprüche 1 bis 9, der zusätzlich ein geeignetes Adjuvans umfaßt.

11. Impfstoff gemäß einem der Ansprüche 1 bis 10 zur Verwendung in der Medizin.

12. Verwendung einer effektiven Menge des kombinierten Impfstoffs gemäß einem der Ansprüche 1 bis 10 in der Herstellung eines Medikaments zur Behandlung eines Menschens, der für eine Infektionserkrankung empfänglich ist oder daran leidet.

## Revendications

1. Vaccin combiné comprenant :
a) un antigène polysaccharidique dérivé de Streptococcus pneumoniae, ou de Neisseria meningitidis A, B ou C conjugué à une protéine porteuse, et
b) la protéine porteuse également présente comme antigène libre,
**caractérisé en ce que** le ratio de polysaccharide sur la protéine porteuse dans le conjugué est de 1:0,3 à 1:2 (p/p).

2. Vaccin combiné selon la revendication 1, dans lequel la protéine porteuse est un antigène diphtérique ou tétanique.

3. Vaccin combiné selon la revendication 2, dans lequel l'antigène diphtérique ou tétanique est l'anatoxine diphtérique ou l'anatoxine tétanique.

4. Vaccin combiné selon la revendication 2, dans lequel l'antigène diphtérique ou tétanique est un dérivé non toxique d'une toxine diphtérique ou tétanique.

5. Vaccin combiné selon l'une quelconque des revendications 1 à 4, dans lequel le ratio polysaccharide:protéine porteuse est de 1:0,5 à 1:1,5 (P/P).

6. Vaccin combiné selon l'une quelconque des revendications 1 à 5, comprenant en outre des antigènes protecteurs contre la diphtérie, le tétanos ou la coqueluche.

7. Vaccin combiné selon l'une quelconque des revendications 1 à 6 comprenant en outre un antigène contre le virus de l'hépatite B.

8. Vaccin combiné selon l'une quelconque des revendications 1 à 7 comprenant en outre un antigène contre le virus de l'hépatite A.

9. Vaccin combiné selon l'une quelconque des revendications 1 à 8 comprenant en outre un antigène assurant une immunité contre la poliomyélite.

10. Vaccin combiné selon l'une quelconque des revendications 1 à 9 comprenant en outre un adjuvant adapté.

11. Vaccin selon l'une quelconque des revendications 1 à 10 à utiliser en médecine.

12. Utilisation d'une quantité efficace du vaccin combiné selon les revendications 1 à 10 dans la fabrication d'un médicament pour le traitement d'un humain susceptible de souffrir d'une maladie infectieuse ou souffrant d'une maladie infectieuse.
